(19) Europäisches Patentamt
European Patent Office
Office européen des brevets

(11) Numéro de publication: 0 121 635
A1

(12) # DEMANDE DE BREVET EUROPEEN

(21) Numéro de dépôt: **83402289.9**

(51) Int. Cl.³: **C 07 H 19/20**, A 61 K 31/70

(22) Date de dépôt: **28.11.83**

(30) Priorité: **03.12.82 FR 8220274**
**14.03.83 FR 8304123**

(43) Date de publication de la demande: **17.10.84**
**Bulletin 84/42**

(84) Etats contractants désignés: **AT BE CH DE FR GB IT LI LU NL SE**

(71) Demandeur: **SYNTHELABO, 58, rue de la Glacière, F-75621 Paris Cedex 13 (FR)**

(72) Inventeur: **Barascut, Jean-Louis, Chemin des Peyridisses, Combailloux F-34980 St Gely Du Fex (FR)**
Inventeur: **Imbach, Jean-Louis Lab. de Chimie Bio-Organique, Place Eugène Bataillon, F-34060 Montpellier Cedex (FR)**
Inventeur: **De Rudder, Jean, 40, Avenue des Etats-Unis, F-78000 Versailles (FR)**

(74) Mandataire: **Thouret-Lemaitre, Elisabeth et al, Service Brevets - SYNTHELABO 58, rue de la Glacière, F-75621 Paris Cedex 13 (FR)**

(54) **3',5'-Diarabinosides, leur préparation et leur application en thérapeutique.**

(57) Dinucléosides monophosphates de formules Ara A-p-Ara C, Ara C-p-Ara A et Ara A-p-Ara A dans lesquelles Ara A est la 9-β-D-arabinofuranosyl-adénine Ara C est la 1-β-D-arabinofuranosyl-cytosine p est le groupe monophosphate. Application en thérapeutique.

EP 0 121 635 A1

# 3',5'-DIARABINOSIDES, LEUR PREPARATION ET LEUR APPLICATION EN THERAPEUTIQUE.

La présente invention a pour objet des 3',5'-diarabinosides, leur préparation et leur application en thérapeutique.

Les nucléosides de l'Arabinose ou Arabinosides, analogues structuraux des Ribosides, présentent d'intéressantes propriétés antivirales et antitumorales qui ont permis l'utilisation en thérapeutique de certains d'entre eux, comme la 9-ß-D-arabinofuranosyl-adénine (Adénine Arabinoside) et la 1-ß-D- arabinofuranosyl-cytosine (Cytosine Arabinoside).

Un inconvénient que l'on rencontre lors de la mise en forme thérapeutique de ces substances résulte de leur faible solubilité (de l'ordre de 1 p. 1000 à la température ordinaire) qui oblige à l'utilisation de solvant tel que le DMSO dans les préparations topiques ou à la perfusion de volumes liquides considérables lorsqu'on doit recourir à la voie générale.

On a cherché à tourner cette difficulté en utilisant à la place des nucléosides leurs 5'-monophosphates qui sont bien solubles. Toutefois ces molécules porteuses de deux charges électriques négatives ne pénètrent que difficilement dans les cellules et, de fait, leur activité antivirale et antitumorale est très faible. Au contraire, les dinucléotides de formule générale Arabinoside-3',5'-phosphate-Arabinoside n'ont qu'une seule charge électrique pour deux molécules de nucléosides et l'expérience montre que leur activité biologique est égale à celle des nucléosides constituants. Leur solubilité est satisfaisante.

Il a été montré précédemment (Privat de Garilhe et De Rudder Progr. Antimicr. & Anticancer Chemother., Vol. II, pp. 180-184, Tokyo 1970) qu'il existe entre Adénine Arabinoside et Cytosine Arabinoside une activité synergique décelable notamment sur la tumeur ascitique d'Ehrlich de la Souris.

Le but de la présente invention est de tirer parti des constatations précédentes en préparant des molécules de 3',5'-diarabinosides dont la solubilité soit satisfaisante et qui soient pourvues d'une très bonne activité.

Les composés de l'invention sont des dinucléotides constitués par les motifs
Ara A = adénine arabinoside ou 9-β-D-arabinofuranosyl-adénine,

Ara C = cytosine arabinoside ou 1-β-D-arabinofuranosyl-cytosine,
et de formules Ara A-p-Ara C, Ara C-p-Ara A et Ara A-p-Ara A p représentant un groupe phosphate.
Les composés de l'invention sont préparés selon les schémas réactionnels suivants :
dans un premier temps on prépare les deux synthons de départ portant un groupe OH libre en 3' (figures 1 et 2) ;
puis dans un deuxième temps on prépare les deux synthons de départ portant un groupe OH libre en 5' (figures 3 et 4) ; et enfin on prépare les dinucléotides monophosphates de l'invention Ara A-p-Ara C, Ara C-p-Ara A et Ara A-p-Ara A (schémas réactionnels 5, 6, 7) par la méthode aux phosphotriesters qui nécessite deux étapes :

. la première étape consiste en une phosphorylation du synthon portant un OH libre en 3' (figure 5)
. la deuxième étape consiste en une condensation du diester ainsi obtenu et du nucléoside à l'aide d'un agent de condensation qui est par exemple le mésitylènesufonyl-1 nitro-3 trazole-1, 2, 4 (MSNT) (figure 6); puis en la déprotection des dimères que l'on obtient finalement sous la forme de sel de triéthylammonium (figure 7).

La phosphorylation du synthon portant un OH libre en 3' peut être effectuée par un agent phosphorylant bifonctionnel tel que l'o-chloro-phényl-phosphorodichloridate.
La réaction des dérivés nucléosidiques protégés en 2' et 5' (6) et (12) avec un excès d'o-chlorophényl phosphorodi-(triazol-1, 2, 4 ide) (19) préparé in situ dans la pyridine conduit, après traitement avec de la triethylamine, aux phosphodiesters (20a) et (20b). Ces derniers ont été isolés sous la forme de sel de triethylammonium après extraction au chloroforme et précipitation dans de l'éther de pétrole (figure 5).

Dans la deuxième étape, l'agent de condensation choisi est le mésitylène sulfonyl-1 nitro-3 trazole-1,2,4 (MSNT) (13).
Une solution, dans de la pyridine des nucléosides phosphodiesters (20a) ou (20b) et des synthons (15) ou (18) est traitée par 2,5 à 3 équivalents de MSNT durant 45 mn à la température ambiante. Le mélange réactionnel brut est ensuite versé dans une solution aqueuse saturée d'hydrogénocarbonate de sodium, et extrait au chloroforme (figure 6).
Les trois dinucléosides monophosphates (21a), (21b) et (21 c) sont obtenus après purification sur colonne de gel de silice et précipitation dans de l'éther de pétrole.
La déprotection des composés (21a), (21b) et (21c) a été effectuée de la manière suivante :

- les groupements O-chlorophényles sont débloqués par une solution de nitro-4 benzaldoxime (0,3M) et de triéthylamine (0,3 M) dans un mélange dioxanne-eau (1/1, V/V) pendant 24 h à 35°C ;
- les groupes benzoyles sont éliminés par l'ammoniaque à 50°C pendant 24 h ;
- le groupe monométhoxytrityle est hydrolysé par de l'acide acétique à 80 % pendant 1 h 15 mn.

Après extraction, les dimères (22a), (22b) et (22c) sont purifiés sur colonne de DEAE-Sephadex A25, puis repurifiés par HPLC semi préparative sur colonne C18 en phase inverse avec un gradient eau/acétonitrile (figure 7).
Ils sont obtenus sous la forme de leurs sels de triéthylammonium.

Les schémas réactionnels sont indiqués ci-dessous.
Les exemples suivants illustrent l'invention.
Les analyses et les spectres UV, IR et RMN confirment la structure des composés.

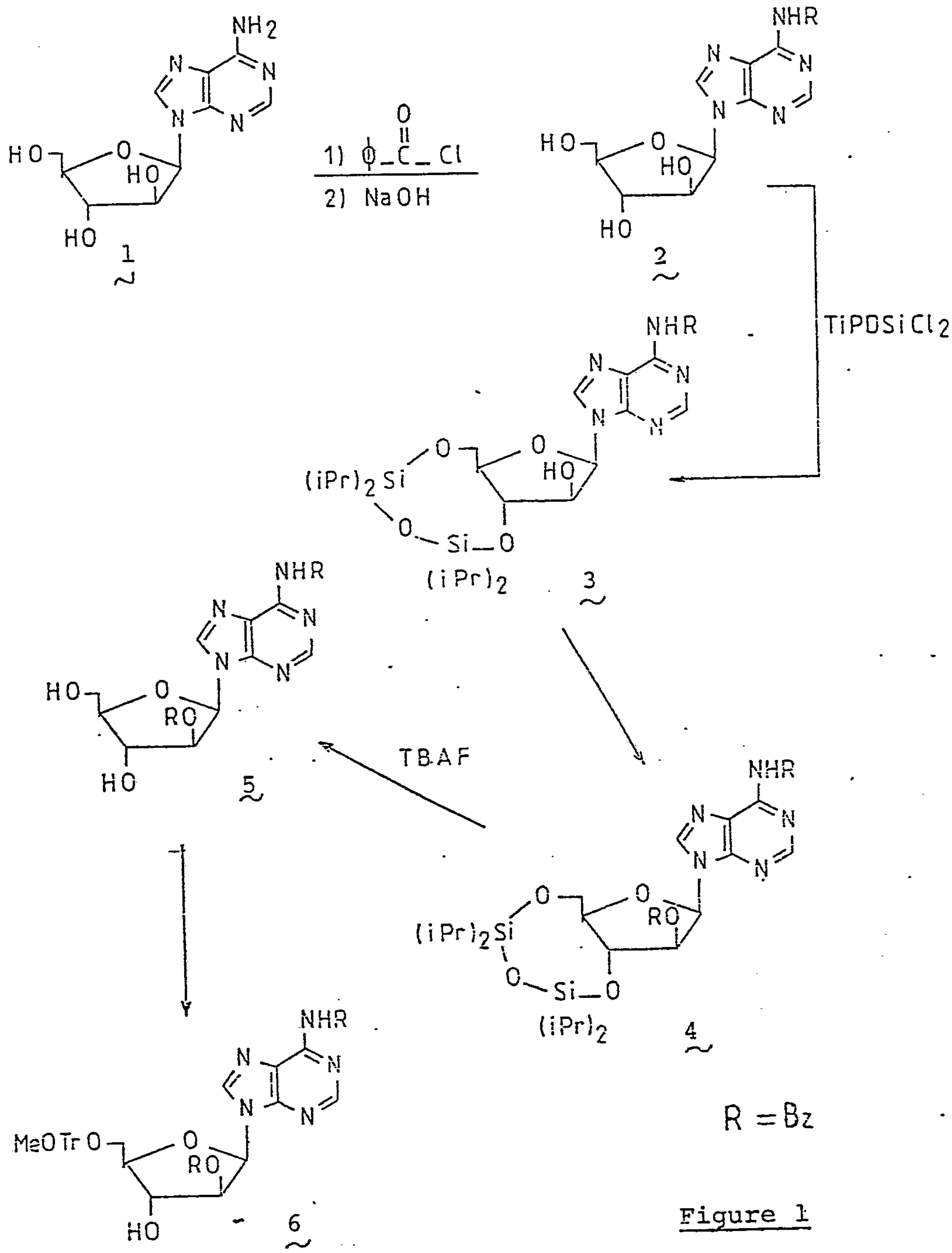


1, 2, 3, 4, 5, 6

R = Bz

Figure 1

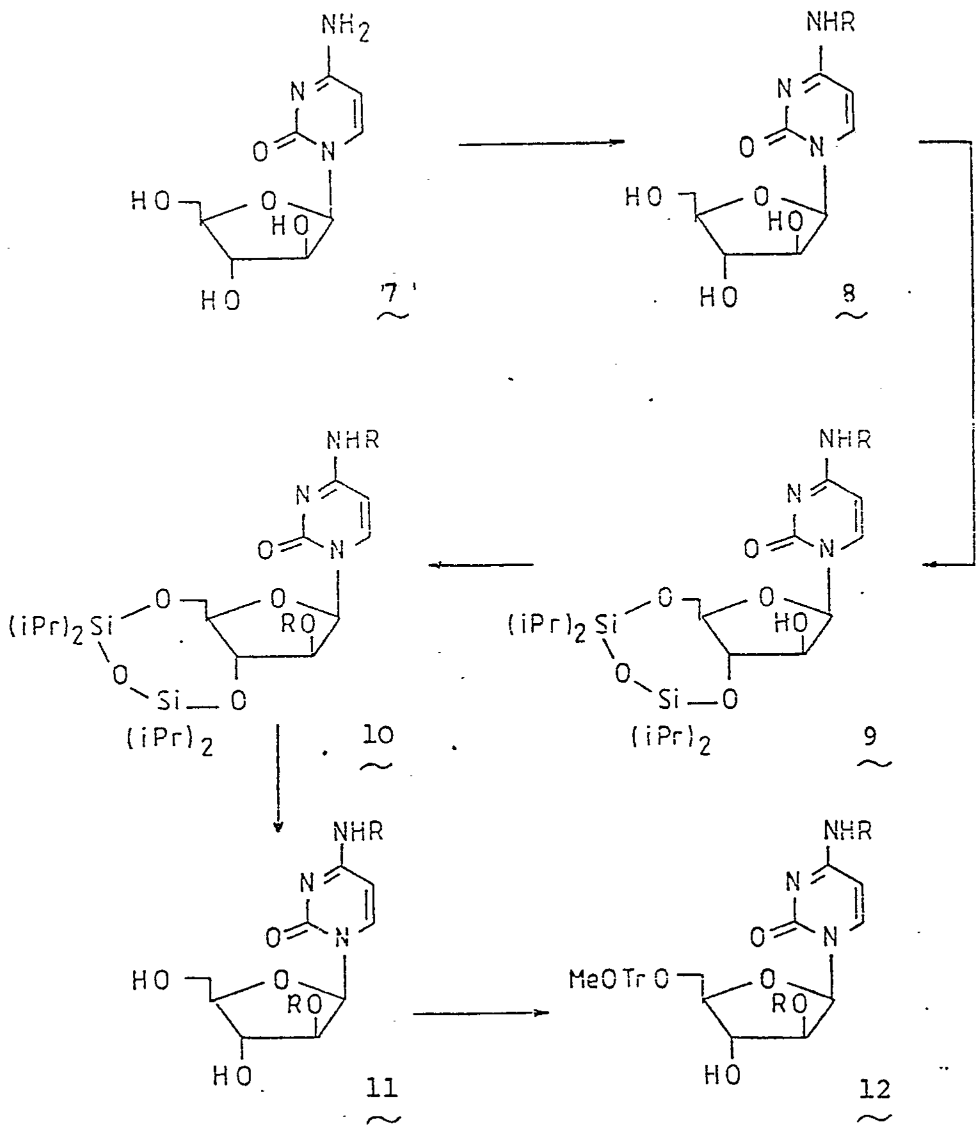

7

8

10

9

11

12

R = Bz

Figure 2

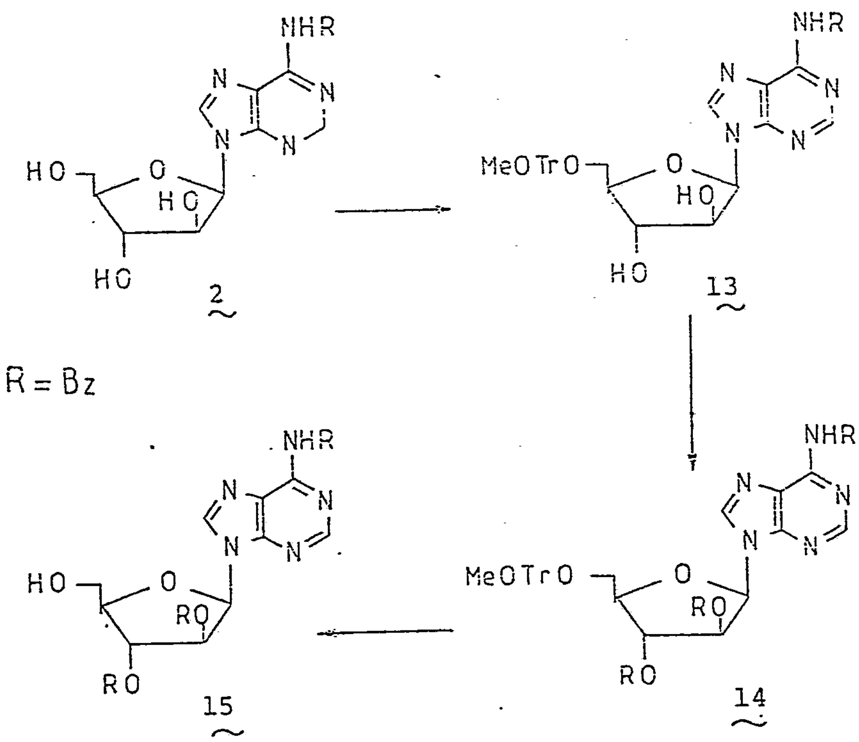

2

13

R = Bz

14

15

Figure 3

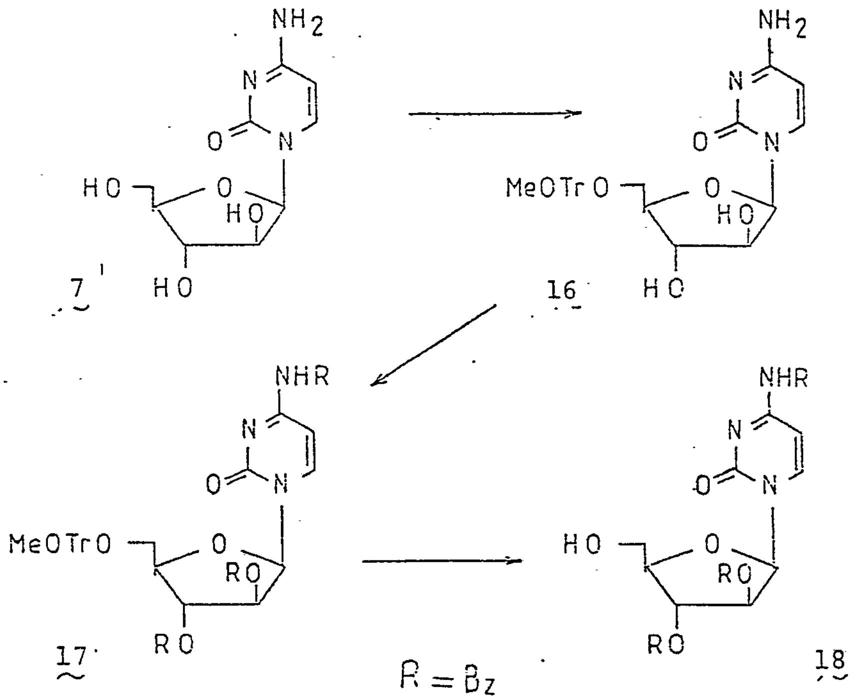

7 16

17 18

R = Bz

Figure 4

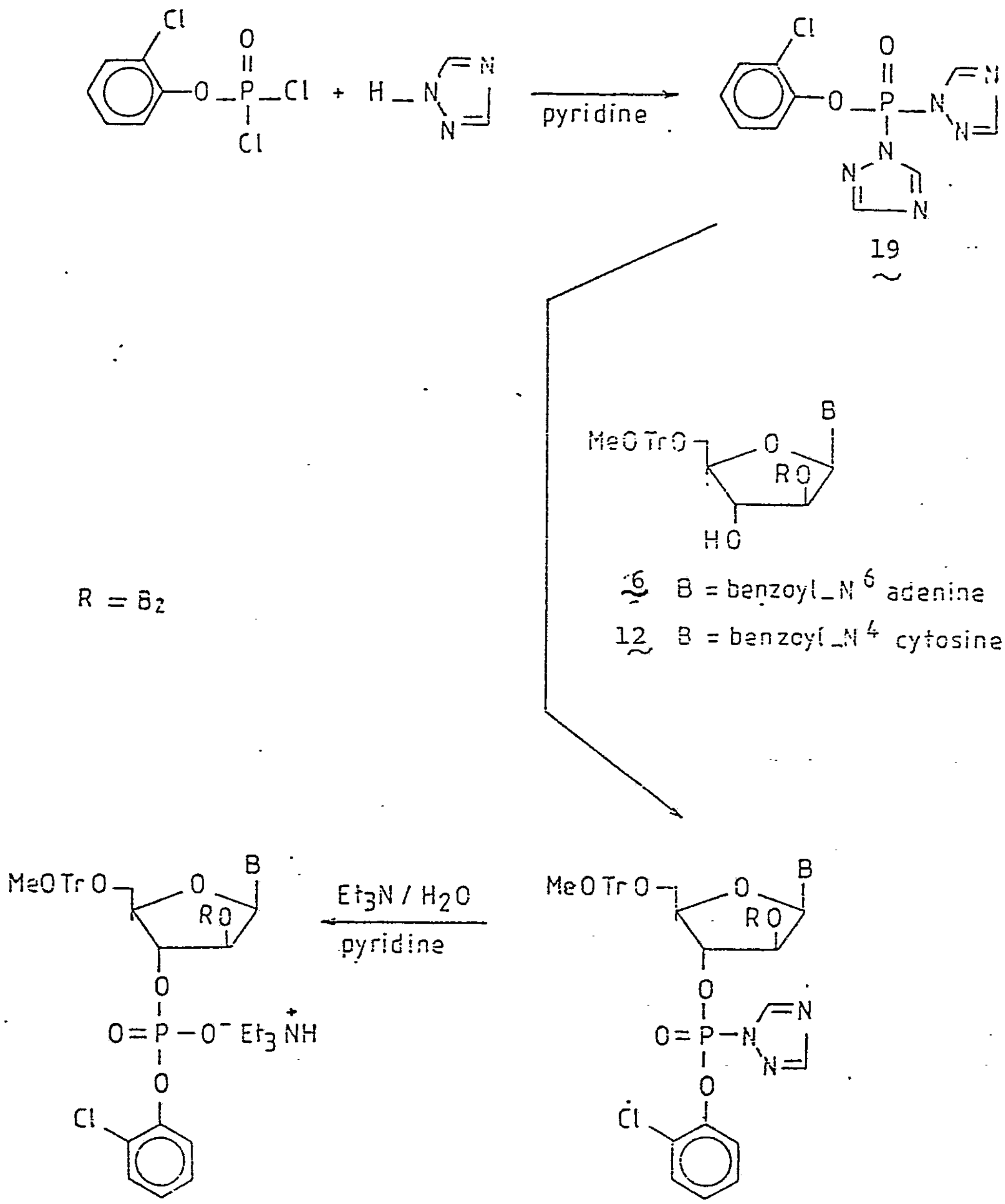


19

R = Bz

6 B = benzoyl-$N^6$ adenine

12 B = benzoyl-$N^4$ cytosine

20a B = benzoyl-$N^6$ adenine

20b B = benzoyl-$N^4$ cytosine

Figure 5

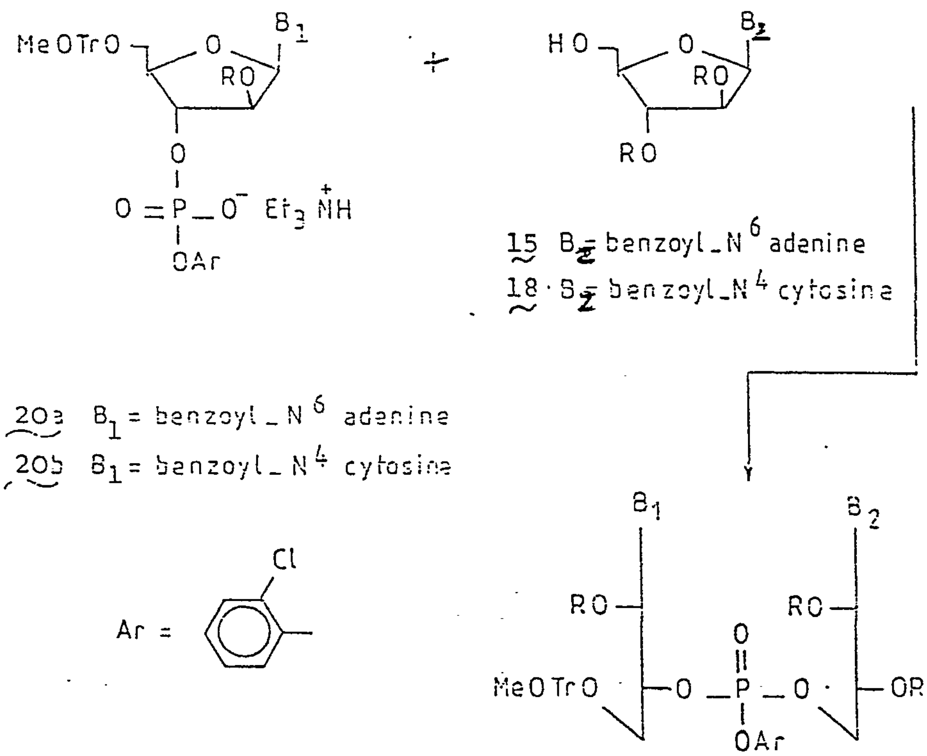

15 $B_2$ = benzoyl-$N^6$ adenine
18 $B_2$ = benzoyl-$N^4$ cytosine

20a $B_1$ = benzoyl-$N^6$ adenine
20b $B_1$ = benzoyl-$N^4$ cytosine

Ar =

21a $B_1$ = benzoyl-$N^6$ adenine ; $B_2$ = benzoyl-$N^4$ cytosine
21b $B_1$ = benzoyl-$N^4$ cytosine ; $B_2$ = benzoyl-$N^6$ adenine
21c $B_1$ = benzoyl-$N^6$ adenine

Figure 6

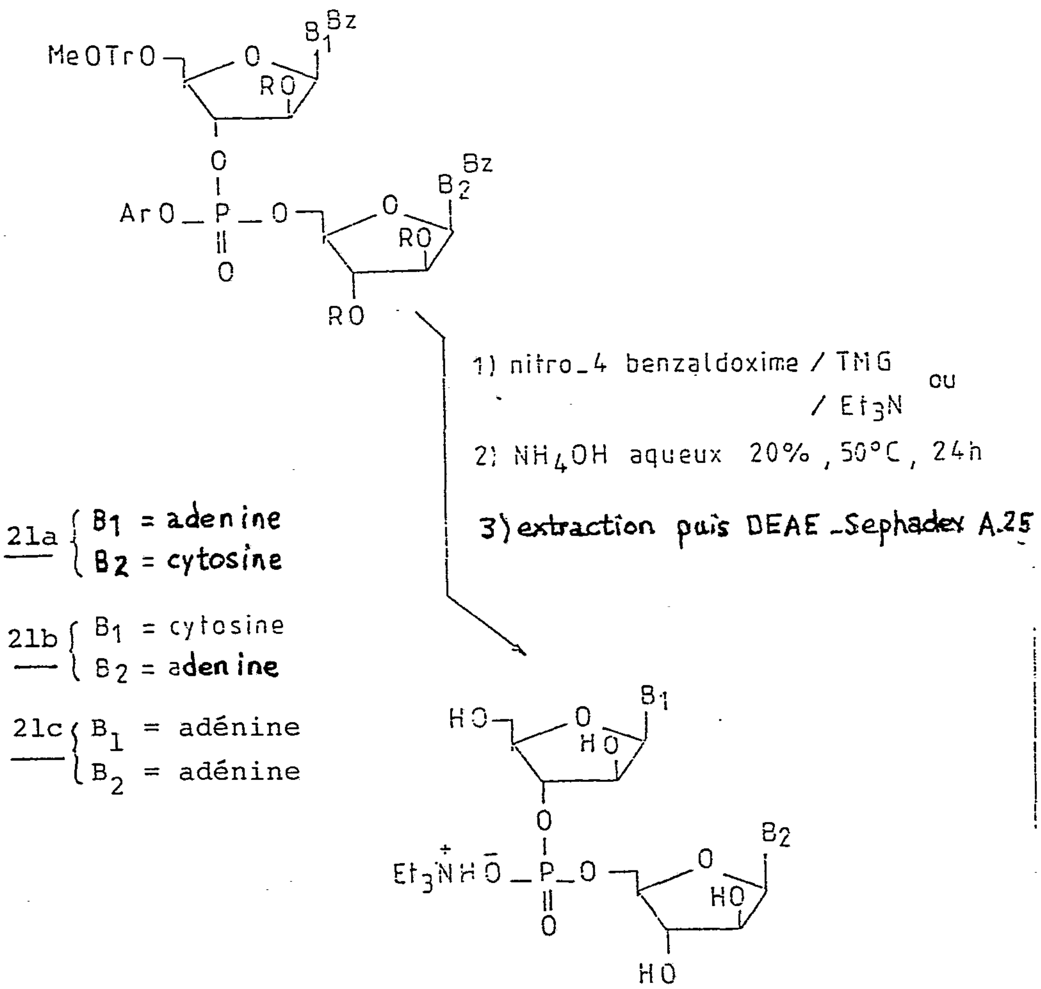


21a { $B_1$ = adenine; $B_2$ = cytosine

21b { $B_1$ = cytosine; $B_2$ = adenine

21c { $B_1$ = adénine; $B_2$ = adénine

22a { $B_1$ = adenine; $B_2$ = cytosine

22b { $B_1$ = cytosine; $B_2$ = adenine

22c { $B_1$ = adénine; $B_2$ = adénine

Figure 7

Exemple 1 : Synthon Ara-adénosine portant un groupe OH libre en 3'.

1.1. benzoyl-$N^6$ β -D- arabinofuranosyl-9 adénine (2)

A une suspension de 1 g (3,74 mmole) d'Arabinosyl-Adénine (1) dans 30 ml de pyridine anhydre , on ajoute goutte à goutte, en refroidissant, 18 ml (150 mmole) de chlorure de benzoyle. Après 36 heures d'agitation à la température ambiante, on ajoute 100 ml d'eau glacée. Le mélange réactionnel est extrait au chloroforme (4 x 60 ml).
La phase organique est lavée à l'hydrogénocarbonate de sodium aqueux (5 x 70 ml), puis à l'eau (5 x 50 ml), séchée sur sulfate de sodium, filtrée, évaporée, ensuite coévaporée au toluène (2 x 50 ml).
Après purification sur colonne de gel de silice (éluant $CHCl_3$), les nucléosides obtenus sont dissous dans 25 ml de pyridine. Cette solution est ajoutée à 40 ml de NaOH (2N), puis agitée durant 20 mn. 80 ml de résine Dowex 50 - $X_2$ (pyridinium) (pH = 6-7) sont ensuite ajoutés. La résine est filtrée, lavée avec (3 x 50 ml) d'eau. La phase aqueuse est extraite à l'éther (6 x 100 ml), concentrée, le précipité formé est filtré, puis cristallisé dans l'eau.On obtient le composé (2)

F = 145 - 147°C (eau)

1.2. TIPDSi-3',5' benzoyl-$N^6$β -D-arabinofuranosyl-9 adénine(3)

A 1,98 g (6,71 mmole) de $TIPDSiCl_2$, on ajoute 1,34 g (19,66 mmole) d'imidazole, 1,66 g (4,47 mmole) du composé (2) et 50 ml de DMF anhydre.
Après une heure d'agitation à la température ambiante, on ajoute 50 ml d'eau. On évapore le mélange sous pression réduite. Le résidu est redissous dans 100 ml d'eau puis extrait au chloroforme (3 x 80 ml). La phase organique est séchée sur sulfate de magnésium, filtrée et évaporée à sec. Après purification sur colonne de gel de silice (éluant $CHCl_3$/AcOEt, 50/50, V/V), on obtient le composé (3) sous la forme d'une mousse blanche.

1.3. TIPDSi-3',5' dibenzoyl-2',$N^6$ β-D- arabinofuranosyl-9 adé-nine (4)

On agite, 3 heures, à la température ambiante une solution contenant 2,1 g (3,42 mmole) du composé (3), 0,851 g (3,76 mmole) d'anhydride benzoïque et 0,138 g (1,14 mmole) de diméthylamino-4 pyridine dans 15 ml de pyridine anhydre.
Après évaporation sous pression réduite de la pyridine, le résidu est dissous dans 100 ml de chloroforme ; la solution résultante est lavée successivement avec une solution saturée d'hydrogénocarbonate de sodium et avec de l'eau (2 x 100 ml). La phase organique est séchée sur sulfate de sodium, filtrée et évaporée sous pression réduite.
Après chromatographie sur colonne de gel de silice (éluant : $CHCl_3$/éther, 98/2, 95/5, 90/10, V/V) le composé (4) est obtenu sous la forme d'une mousse blanche.

1.4. Di-benzoyl-2',$N^6$ β-D- arabinofuranosyl-9 adenine (5)

On dissout 100 mg (0,139 mmole) du composé (4) dans 6 ml d'une solution (0,5M) de fluorure de tétrabutylammonium dans du tétrahydrofuranne ; après 45 mn d'agitation à la température ambiante, la solution est évaporée sous pression réduite. Le résidu est chromatographié sur colonne de gel de silice (éluant $CHCl_3$/MeOH, 99/1, 98/2, V/V/). Le composé (5) est obtenu après précipitation dans le chloroforme.

F = 124 - 126°C ($CHCl_3$)

1.5. Monomethoxytrityl-5'di-benzoyl-2',$N^6$ β-D- arabinofuranosyl-9 adenine (6)

Une solution contenant 0,900 g (1,89 mmole) du composé (5) et 1,10 g (3,78 mmole) de chlorure de monométhoxytrityle dans 20 ml de pyridine anhydre est agitée durant 22 heures à la température ambiante à l'abri de l'humidité et de la lumière. Après addition de 150 ml d'eau glacée, le mélange réactionnel est extrait avec du chloroforme (3 x 70 ml). La phase organique est séchée sur sulfate de sodium, filtrée, évaporée à sec,

puis coévaporée au toluène (2 x 30 ml). Le résidu est chromatographié sur colonne de gel de silice (éluant : $CHCl_3/Et_3N$ 100/0,1 , V/V). Le produit (6) est obtenu après précipitation dans de l'éther de pétrole.

Exemple 2 : Synthon Ara-cytidine portant un groupe OH libre en 3'.

2.1. Benzoyl-$N^4$β-D- arabinofuranosyl-1 cytosine (8)

A une suspension de 500 mg (1,79 mmole) de chlorhydrate d'Ara-C dans 5 ml de méthanol anhydride on ajoute 10 ml (1,79 mmole de sodium) de méthylate de sodium. Après 15 mn d'agitation à la température ambiante, le résidu est évaporé, coévaporé à l'éthanol 100, puis à la pyridine anhydre. Au précipité blanc obtenu, on ajoute 10 ml de pyridine et 8 ml de chlorure de benzoyle (à 0°C). Après une nuit d'agitation, le mélange est versé sur 50 ml d'eau glacée, puis extrait au chloroforme (3 x 50 ml). La phase organique est lavée à l'eau (3 x 30 ml), à l'hydrogénocarbonate de sodium, filtrée et évaporée au toluène (2 x 10 ml). Le mélange réactionnel est purifié sur colonne de gel de silice (éluant $CHCl_3$). La mousse obtenue est dissoute dans 22 ml de pyridine puis additionnée de 11 ml de NaOH(2N). Après 20 mn d'agitation, la solution est neutralisée par la résine Dowex-50 W $X_2$ (pyridinium). La résine est filtrée, lavée à l'eau (3 x 80 ml). La phase aqueuse est extraite à l'éther (5 x 100 ml), puis évaporée sous pression réduite. Le produit (8) cristallise dans le méthanol.

F = 186 - 190°C (méthanol)

2.2. TIPDSi-3',5' benzoyl-$N^4$β-D- arabinofuranosyl-1 cytosine (9)

On dissout 200 mg (0,82 mmole) du composé (8) dans 4 ml de pyridine. A cette solution on ajoute 0,3 ml (0,97 mmole) de $TIPDSiCl_2$. Après une heure d'agitation à la température ambiante, le mélange est évaporé à sec. Le résidu obtenu est redissous dans 10 ml de chloroforme, lavé avec 20 ml d'eau,

et extrait avec (3 x 20 ml) de chloroforme. Les phases organiques sont rassemblées, séchées sur sulfate de magnésium, filtrées et évaporées sous pression réduite. L'huile résultante est fractionnée sur colonne de gel de silice (éluant : $CHCl_3$, $CHCl_3$/MeOH, 99/1, V/V). On obtient le composé (9).

2.3. TIPDSi-3',5' dibenzoyl-2',$N^4$β-D- arabinofuranosyl-1 cytosine (10)

On agite une solution de 250 mg (0,42 mmole) du composé (9), 105 mg (0,46 mmole) d'anhydride benzoïque et 17 mg (0,14 mmole) de diméthylamino-4 pyridine, dans 2 ml de pyridine anhydre, à la température ambiante, pendant deux heures. Le mélange réactionnel brut est versé sur 10 ml d'eau, puis extrait au chloroforme (3 x 10 ml). La phase organique est lavée à l'eau (3 x 10 ml), séchée sur sulfate de magnésium, filtrée, évaporée, puis coévaporée au toluène (2 x 5 ml). Le résidu est fractionné sur colonne de gel de silice (éluant $CHCl_3$/MeOH 99,5/0,5 , V/V).

Le composé (10) est obtenu sous la forme d'une mousse blanche.

2.4. Dibenzoyl-2',$N^4$β-D- arabinofuranosyl-1 cytosine (11)

On dissout 245 mg (0,35 mmole) du composé (10) dans 6,5 ml d'une solution (0,5 M) de fluorure de tétrabutylammonium dans du THF anhydre. Après 45 mn d'agitation à la température ambiante, la solution est évaporée sous pression réduite. Le résidu est chromatographié sur colonne de gel de silice (éluant $CHCl_3$/MeOH, 98/2, V/V). On obtient le composé (11).

F = 219 - 222°C (chloroforme)

2.5. Monomethoxytrityl-5' dibenzoyl-2',$N^4$β-D- arabinofuranosyl-1 cytosine (12)

On ajoute une solution de 140 mg (0,31 mmole) du composé (11) anhydre et 184 mg (0,62 mmole) de chlorure de monométhoxytrityle dans 2 ml de pyridine anhydre, durant 22 heures, à la température ambiante, à l'abri de l'humidité et de la lumière.

Après addition de 10 ml d'eau glacée, on extrait le mélange réactionnel au chloroforme (3 x 10 ml). La phase organique est séchée sur sulfate de magnésium, filtrée, évaporée, puis coévaporée au toluène (2 x 5 ml). L'huile obtenue est chromatographiée sur colonne de gel de silice (éluant : $CHCl_3$/MeOH/$Et_3N$, 99/1/0,1, V/V/V). On obtient le composé (12).

Exemple 3 Synthon Ara-adénosine portant un groupe OH libre en 5'.

3.1. Monomethoxytrityl-5' benzoyl-$N^6$β-D- arabinofuranosyl-9 adenine (13)

On agite une solution de 400 mg (1,08 mmole) du composé (2) et 650 mg (2,16 mmole) de chlorure de monométhoxytrityle dans 7 ml de pyridine anhydre, à la température ambiante, durant 22 heures, à l'abri de l'humidité et de la lumière. Après addition de 25 ml d'eau glacée, le mélange réactionnel est extrait au chloroforme (3 x 15 ml). La phase organique est séchée sur sulfate de sodium, filtrée, évaporée, puis coévaporée au toluène (2 x 10 ml).
Le résidu est chromatographié sur colonne de gel de silice (éluant $CHCl_3$/MeOH/$Et_3N$, 98/1/1 , V/V/V). On obtient le composé (13).

3.2. Monomethoxytrityl-5' tribenzoyl-2',3',$N^6$β-D- arabinofuranosyl-9 adénine (14)

On agite une solution de 200 mg (0,31 mmole) du composé (13) de 146 mg (0,69 mmole) d'anhydride benzoïque et de 13 mg (0,10 mmole) de diméthylamino-4 pyridine dans 2 ml de pyridine anhydre, à la température ambiante, durant 1 h 15.
Après addition de 30 ml d'hydrogénocarbonate de sodium aqueux, la solution est extraite au chloroforme (3 x 20 ml). La phase organique est lavée à l'eau (3 x 15 ml), séchée sur sulfate de sodium, filtrée, évaporée, coévaporée au toluène (2 x 10 ml).
Le résidu est purifié sur colonne de gel de silice (éluant $CHCl_3$/hexane/$Et_3N$, 80/19/1 ; V/V/V).
Le compose (14) est obtenu après précipitation dans de l'hexane.

3.3. Tribenzoyl-2',3',$N^6$ß-D- arabinofuranosyl-9 adénine (15)

On dissout 200 mg (0,24 mmole) du composé (14) dans 9 ml d'un mélange chloroforme/méthanol (7/3, V/V ) contenant 2 % d'acide benzène-sulfonique. On agite le mélange réactionnel pendant 20 mn à 0°C. On ajoute 10 ml d'hydrogénocarbonate de sodium aqueux. Le mélange est extrait au chloroforme (3 x 8 ml). La phase organique est lavée avec 10 ml d'hydrogénocarbonate de sodium aqueux, puis avec de l'eau (3 x 10 ml), séchée sur sulfate de sodium, filtrée et évaporée sous pression réduite. Le résidu est purifié sur colonne de gel de silice (éluant $CHCl_3$/MeOH, 99/1, V/V).
Le produit (15) est obtenu après cristallisation dans de l'éthanol.

F = 135 - 137°C (éthanol)

Exemple 4 Synthon Ara-cytidine portant un groupe OH libre en 5'.

4.1. Monométhoxytrityl-5'ß-D- arabinofuranosyl-1 cytosine (16)

On agite le mélange contenant 100 mg (0,41 mmole) d'Ara-C(7) et 186 mg (0,61 mmole) de chlorure de monométhoxytrityle dans 3 ml de pyridine anhydre est agitée à température ambiante durant 10 heures à l'abri de l'humidité et de la lumière. Après addition de 10 ml d'eau glacée, le mélange réactionnel est extrait avec (3 x 10 ml) de chloroforme ; la phase organique est séchée sur sulfate de magnésium, filtrée, évaporée, puis coévaporée au toluène (2 x 10 ml). Le résidu est chromatographié sur colonne de gel de silice (éluant : $CHCl_3$/MeOH/$Et_3N$, 95/5/0,1, V/V/V) on obtient le composé(16).

4.2. Monomethoxytrityl-5' tribenzoyl-2',3',$N^4$ß-D- arabinofuranosyl-1 cytosine(17).

Une solution contenant 415 mg (0,82 mmole) du composé(16), 615 mg (2,72 mmole) d'anhydride benzoïque, 34 mg (0,27 mmole) de diméthylamino-4 pyridine, dans 3 ml de pyridine, est agitée une nuit à la température ambiante.

20 ml d'eau sont ajoutés. La solution résultante est extraite au chloroforme (3 x 20 ml). La phase organique est séchée sur sulfate de magnésium, filtrée, évaporée, puis coévaporée au toluène. Le résidu est chromatographié sur colonne de gel de silice (éluant $CHCl_3$/hexane/$Et_3N$, 79/20/1, V/V/V). On obtient le composé (17).

4.3. Tribenzoyl-2',3'$N^4$ß-D- arabinofuranosyl-1 cytosine (18)

On dissout 672 mg (0,82 mmole) du composé (17) dans 20 ml d'un mélange chloroforme/méthanol (7/3, V/V/) contenant 2 % d'acide benzène-sulfonique, la réaction est agitée pendant 20 mn à 0°C. 20 ml d'hydrogénocarbonate de sodium aqueux sont additionnés, le mélange est extrait au chloroforme (3 x 20 ml). La phase organique est séchée sur sulfate de magnésium, filtrée, évaporée sous pression réduite. Le résidu est fractionné sur colonne de gel de silice (éluant : $CHCl_3$/MeOH, 98,5/1,5, V/V). Le produit (18) est obtenu après cristallisation dans de l'éthanol à 95 %.

F = 169 - 171°C (éthanol 95)

Exemple 5 o-Chlorophenylphosphate-3' (monométhoxytrityl-5' dibenzoyl-2',$N^6$ Ara-A) de triéthylammonium (20 a)

On prépare, in situ, une solution d'o-chlorophénylphosphorodi-(triazol-1, 2,4 ide) (12) dans 1 ml de pyridine anhydre, à partir de 100 mg (0,408 mmole) d'o-chlorophénylphosphorodichloridate et de 162 mg (2,347 mmole) de triazole-1,2,4. Après 20 minutes d'agitation à la température ambiante, 80 mg (0,107 mmole) du composé (6) sont ajoutés. L'agitation est poursuivie durant 20 mn. On ajoute ensuite une solution composée de 0,75 ml de triéthylamine, 0,5 ml d'eau et 0,5 ml de pyridine. La réaction est arrêtée 10 mn après, en ajoutant 8 ml d'hydrogénocarbonate de sodium aqueux. Le mélange est extrait avec du chloroforme (3 x 8 ml).

La phase organique est séchée sur sulfate de magnésium, filtrée, évaporée, coévaporée au toluène (2 x 5 ml).

Après purification sur colonne de gel de silice (éluant : $CHCl_3/MeOH/Et_3N$, 98/2/0,1 V/V/V) le composé (20 a) est obtenu après précipitation dans de l'éther de pétrole.

Rf = 0,11 (éluant $CHCl_3/MeOH$, 90/10, V/V )

U.V. : $\lambda_{max}$ (éthanol 95) : 278 nm ($\varepsilon$ = 20 700) 232 nm ($\varepsilon$ = 33 500)

Dimère Ara A-p-Ara C (21a) complètement protégé

A une solution de 601 mg (0,578 mmole) du phosphodiester (20a) et de 305 mg (0,549 mmole) du tribenzoyl-2',3',$N^4$ Ara-C (18) dans 2,9 ml de pyridine, on ajoute 513 mg (1,735 mmole) de mésitylène sulfonyl-1 nitro-3 triazole-1,2,4. La réaction est arrêtée après 45 mn d'agitation à la température ambiante, en ajoutant 0,8 ml d'hydrogénocarbonate de sodium. Après 10 mn, la solution obtenue est versée sur 20 ml d'hydrogénocarbonate de sodium aqueux, puis extraite au chloroforme (4 x 20 ml). La phase organique est séchée sur sulfate de magnésium, filtrée, évaporée, et coévaporée au toluène (2 x 10 ml). Le résidu est fractionné sur colonne de gel de silice (éluant : $CHCl_3/Et_3N$ 100/0,1, V/V ). Le dimère (21a) est obtenu après précipitation dans l'éther de pétrole.

Rf = 0,20 ; 0,34 deux diastéréoisomères (éluant : $CHCl_3/MeOH$ ; 97/3 ; V/V )

U.V. : $\lambda_{max}$ (éthanol 95) : 269 nm ($\varepsilon$ = 11 000) 234 nm ($\varepsilon$ = 20 000)

Exemple 6 o-Chlorophenylphosphate-3' (monométhoxytrityl-5' dibenzoyl-2',$N^4$ AraC) de triéthylammonium(20 b).

On prépare, in situ, une solution d'o-chlorophénylphosphorodi-(triazol-1,2,4 ide) (12) dans 2 ml de pyridine anhydre, à partir de 272 mg (1,11 mmole) d'o-chlorophénylphosphorodichloridate, de 377 mg (5,54 mmole) de triazole-1,2,4. Après 20 mn d'agitation à température ambiante, 160 mg (0,22 mmole) du composé (12) sont ajoutés. L'agitation est poursuivie durant 20 mn. On ajoute ensuite une solution composée de 0,5 ml de triéthylamine, 0,3 ml d'eau et 0,3 ml de pyridine. La réaction est arrêtée 10 mn après. Le mélange réactionnel est traité suivant le même mode opératoire que lors de la synthèse de (20 a). Le résidu est chromatographié sur colonne de gel de silice (éluant : $CHCl_3/MeOH/Et_3N$, 97/3/0,1, V/V/V). Le composé (20 b) est obtenu après précipitation dans de l'éther de pétrole.

Rf = 0,23 (éluant : $CHCl_3/MeOH$, 90/10, V/V )

U.V. : $\lambda_{max}$ (éthanol 95) : 303 nm ($\varepsilon$= 12 300)
261 nm ( = 31 700)
232 nm ($\varepsilon$= 54 100)

Dimère Ara C-p-Ara A(21 b) complètement protégé

A une solution de 200 mg (0,197 mmole) du phosphodiester (20b) et de 110 mg (0,191 mmole) du tribenzoyl-2',3',$N^6$ Ara A(15) dans 1 ml de pyridine, on ajoute 175 mg (0,591 mmole) de mésitylène sulfonyl-1 nitro-3 triazole-1,2,4. La réaction est arrêtée après 45 mn d'agitation à température ambiante, puis traitée comme ci-dessus. Le résidu obtenu est purifié sur colonne de gel de silice (éluant Benzène/MeOH/$Et_3N$, 99/0,9/0,1, V/V/V). Le dimère (21b) est obtenu après précipitation dans de l'éther de pétrole.

Rf= 0,20; deux diastéréoisomères (éluant : $CHCl_3/MeOH$, 97/3 V/V)
U.V. : $\lambda_{max}$ (éthanol 95) : 269 nm ($\varepsilon$= 9 700) 232 nm ($\varepsilon$= 17 600)

Exemple 7 o-Chlorophenylphosphate-3' (monométhoxytrityl-5' dibenzoyl-2',$N^6$ Ara-A) de triéthylammonium(20 c)

On prépare, in situ, une solution d'o-chlorophénylphosphorodi-(triazol-1, 2,4 ide) (12) dans 1 ml de pyridine anhydre, à partir de 100 mg (0,408 mmole) d'o-chlorophénylphosphorodichloridate et de 162 mg (2,347 mmole) de triazole-1,2,4. Après 20 minutes d'agitation à la température ambiante, 80 mg (0,107 mmole) du composé(6)sont ajoutés. L'agitation est poursuivie durant 20 mn. On ajoute ensuite une solution composée de 0,75 ml de triéthylamine, 0,5 ml d'eau et 0,5 ml de pyridine. La réaction est arrêtée 10 mn après, en ajoutant 8 ml d'hydrogénocarbonate de sodium aqueux. Le mélange est extrait avec du chloroforme (3 x 8 ml).

La phase organique est séchée sur sulfate de magnésium, filtrée, évaporée, coévaporée au toluène (2 x 5 ml).

Après purification sur colonne de gel de silice (éluant : $CHCl_3/MeOH/Et_3N$, 98/2/0,1 V/V/V) le composé(20 a)est obtenu après précipitation dans de l'éther de pétrole.

Rf = 0,11 (éluant $CHCl_3/MeOH$, 90/10, V/V )

U.V. : $\lambda_{max}$ (éthanol 95) : 278 nm ($\varepsilon$ = 20 700) 232 nm ($\varepsilon$ = 33 500)

Dimère Ara A p Ara A(21a)complètement protégé

A une solution de 106 mg (0,103 mmole) du phosphodiester(20a) et de 58 mg (0,100 mmole) du tribenzoyl-2',3',$N^6$ Ara-A(15) dans 0,5 ml de pyridine, on ajoute 91 mg (0,307 mmole) de mésitylène sulfonyl-1 nitro-3 triazole-1,2,4. La réaction est arrêtée après 45 mn d'agitation à la température ambiante, en ajoutant 0,2 ml d'hydrogénocarbonate de sodium. Après 10 mn, la solution obtenue est versée sur 12 ml d'hydrogénocarbonate de

sodium aqueux, puis extraite au chloroforme (4 x 10 ml). La phase organique est séchée sur sulfate de magnésium, filtrée, évaporée, et coévaporée au toluène (2 x 5 ml). Le résidu est fractionné sur colonne de gel de silice (éluant : $CHCl_3$/MeOH/pyridine 99/1/0,1 V/V/V). Le dimère (21a) est obtenu après précipitation dans de l'éther de pétrole.

Rf = 0,57 deux diastéréoisomères (éluant $CHCl_3$/MeOH 95/5 V/V)

Exemple 8 : Déprotection et purification des dimères AraA p Ara C (21a) et Ara C p Ara A (21b).

a) Déprotection

Une solution de 0,020 mmole d'oligonucléotide totalement protégé (21 a ou 21b) de (0,3 M de nitro-4 syn-benzaldoxime et de 0,3 M de triéthylamine, dans un mélange dioxanne - eau (4 ml ; 1/1 ; V/V ) est agitée,24 heures,à 30°C.
Après évaporation sous pression réduite on ajoute à la gomme obtenue 5,4 ml d'ammoniaque concentrée(20 %) ; cette solution est chauffée 24 heures à 50°C dans un tube scellé.
Le mélange réactionnel brut est neutralisé par la résine Dower 50 W-$X_2$ (forme pyridinium)La résine est filtrée, lavée à l'eau (3 x 20 ml) et à EtOH (3 x 20 ml). La phase eau-éthanol est évaporée à sec. Le résidu est dissous dans 40 ml d'eau. La phase aqueuse est lavée à l'éther (10 x 30 ml), puis évaporée sous pression réduite. Au résidu obtenu, on ajoute 4 ml d'acide acétique aqueux à 80 %. La solution ainsi obtenue est agitée à température ambiante durant 1 h 15, puis évaporée, coévaporée avec de l'eau jusqu'à pH neutre, puis successivement lavée avec du chloroforme (15 x 30 ml) et de l'éther éthylique (5 x 20 ml).
La phase aqueuse est évaporée sous pression réduite.

b) Purification

Le résidu est purifié sur colonne de DEAE Sephadex A-25 (gradient linéaire $10^{-3}$M à 0,3M, tampon aqueux d'hydrogéno-carbonate de triéthylammonium, pH = 7,5).

Les fractions contenant le dimère(21) sont réunies et évaporées. Le produit ainsi obtenu est repurifié par HPLC semi-préparative. Celle-ci est réalisée sur colonne $C_{18}$ avec un gradient eau, $CH_3CN$ (5 %) d'une durée de 13 mn.
Les fractions contenant le dimère(21) sont évaporées à sec, coévaporées à l'eau, puis lyophilisées.
On obtient ainsi les sels de triéthylammonium (22a) et (22b) des dimères Ara A-p-Ara C et Ara C-p-Ara A.

Exemple 9 : Déprotection et purification du dimère Ara A-p-Ara A (21c).

1 g du mélange (dimère 21 c + impureté) est coévaporé à la pyridine anhydre (3 x 20 ml), puis dissous dans 5 ml de pyridine ; on ajoute 2 ml de chlorure de benzoyle. Après 24 heures d'agitation, on ajoute 100 ml d'eau, on extrait le mélange au chloroforme (5 x 20 ml). La phase organique est lavée à l'eau (5 x 10 ml), à l'hydrogénocarbonate de sodium aqueux (5 x 10 ml), à l'eau (5 x 10 ml), séchée sur sulfate de magnésium, filtrée, évaporée, coévaporée au toluène, puis au méthanol anhydre. Le résidu est dissous dans 15 ml de méthanol, puis additionné de 6 ml (0,92 M) de méthylate de sodium. Après 4 heures d'agitation, on neutralise avec une résine Dowex 50 W-$X_2$ (pyridinium), la résine est filtrée, lavée à l'eau (3 x 50 ml). La solution est évaporée sous pression réduite. La gomme obtenue est solubilisée dans 20 ml d'eau, lavée au chloroforme (15 x 20 ml), à l'éther (10 x 20 ml).
Après purification sur DEAE-sephadex A-25, avec un gradient linéaire ($10^{-3}$M à 0,3M) en hydrogénocarbonate de triéthylammonium (pH = 7,5), les fractions contenant le dimère 21a sont repurifiées par HPLC semi préparative sur colonne de $C_{18}$ avec un gradient linéaire ($CH_3CN$ 4 % à 20 %, 15 mn, débit 10 ml/mn) évaporées, coévaporées à l'eau, puis lyophilisées.
On obtient ainsi le sel de triéthylammonium (22c) du dimère AraA-p-Ara A.

Les composés de l'invention ont été soumis à des essais pharmacologiques montrant leur intérêt dans le domaine antitumoral. L'action des composés sur le développement de la tumeur ascitique d'Ehrlich chez la Souris a été observée.
Les essais se font sur des lots de 5 ou 6 souris Swiss de 12 à 15 g. Chaque animal reçoit 2 x $10^6$ cellules malignes par voie péritonéale. Le traitement débute 16 h après l'inoculation et consiste en 2 injections par jour de 0,75 ml d'une solution de dinucléotide à 2 mg/ml dans le péritoine c'est-à-dire 3 mg par animal et par jour ou 200 mg/kg. La durée du traitement est de 4 jours.

Les dinucléotides, composés de l'invention, ont été testés de la manière indiquée.
Les résultats sont donnés dans les tableaux suivants :

TABLEAU 1

| | Témoins | Ara A | Ara C | Témoins | Ara A-p-Ara C |
|---|---|---|---|---|---|
| Durée de survie des souris | 16,54 j | 24,5 j | 32 j | 18 j | > 36 j |
| % d'augmentation de survie (pour les animaux décédés) | - | 48,5% | 94,4% | - | > 100% |
| Nombre d'animaux sans tumeur au 28ème jour | 0/12 | 0 | 0 | 0/5 | 4/5 |

Tableau 2

| | Témoins | Ara A | Ara A-p-Ara A |
|---|---|---|---|
| Durée de survie des Souris | 14,67 j | 20,5 j | 21,17 j |
| % d'augmentation | - | 39,75 % | 44,3 % |

L'activité des trois composés de l'invention Ara A-p-Ara A, Ara A-p-Ara C et Ara C-p-Ara A a également été démontrée sur le virus Herpès.

La récolte virale témoin (cellules non traitées) a un titre TCI $D_{50}$ (tissue culture infecting dosis) de $10^{5,625}$.

Les cultures traitées par les composés de l'invention ont les titres suivants

Ara A-p-Ara A $TCID_{50} = 10^{3,16}$

Ara A-p-Ara C $TCID_{50} < 10^{2}$

Ara C-p-Ara A $TCID_{50} \leqslant 10^{2,5}$

Les composés de l'invention possèdent des propriétés antitumorales et peuvent être utilisés pour le traitement de diverses maladies telles que

- certaines affections virales causées par des virus à ADN :
  . Herpès dans toutes ses localisations, herpès 1 et herpès 2,
  . Varicelle
  . Zona
  . Cytomégalie
  . Mononucléose infectieuse, tumeur de Burkitt (virus d'Epstein Barr)
  . Hépatite B
  . Maladies du groupe Variole-Vaccine
  . Adénoviroses
  . Retroviroses

- certaines affections malignes sensibles à Ara A (leucémies myéloïdes chroniques en cours de poussées aiguës) ou à Ara C (leucose myéloblastique aiguë).

Les compositions pharmaceutiques de l'invention peuvent être liquides ou solides et se présenter sous la forme de solutions, collyres, onguents, pommades...

Elles sont préparées selon les méthodes usuelles. Le principe actif peut y être incorporé à des excipients pharmaceutiques habituellement utilisés dans de telles compositions et choisis en fonction de la forme galénique à réaliser. Ces excipients peuvent être constitués par des solvants aqueux ou non, des stabilisants, des mouillants, des vaselines, des lanolines, des polyéthylène -glycols, des esters d'acides gras du sorbitanne, polyhydroxyéthylés ou non.

La concentration en principe actif des préparations pharmaceutiques ainsi réalisées est variable selon la gravité des cas et le mode d'utilisation envisagé : elle peut être par exemple comprise entre 0,1 % et 5 %.

Lorsque les composés de l'invention sont utilisés par voie externe, sous la forme de pommades ou d'onguents, la concentration en composé va de 1 à 10 %, de préférence elle est de 3 %. On renouvelle les applications sur la peau ou les muqueuses toutes les 3 heures environ.

Lorsque les composés de l'invention sont utilisés par voie intraveineuse, sous forme de perfusion ou d'injection, ils le sont à raison de 15 à 25 mg/kg de poids corporel par jour.

## Revendications

1. Dinucléosides monophosphates de formules Ara A-p-Ara C, Ara C-p-Ara A et Ara A-p-Ara A dans lesquelles

Ara A est la 9-β-D-arabinofuranosyl-adénine

Ara C est la 1-β-D-arabinofuranosyl-cytosine

p est le groupe monophosphate.

2. Procédé de préparation des composés selon la revendication 1, procédé caractérisé en ce que

1) on prépare les synthons adénine et cytosine portant un groupe OH libre en 3',

2) on prépare les synthons adénine et cytosine portant un groupe OH libre en 5',

3) on effectue la synthèse des dinucléosides monophosphates

- en phosphorylant le synthon portant un OH libre en 3' à l'aide d'un agent phosphorylant bifonctionnel,
- en condensant le diester obtenu avec le nucléoside choisi à l'aide d'un agent de condensation,
- et en déprotégeant les composés obtenus pour arriver aux dimères cherchés sous la forme de sels de triéthylammonium.

3. Procédé selon la revendication 2, caractérisé en ce que l'agent phosphorylant bifonctionnel est l'o-chloro-phényl phosphorodichloridate.

4. Procédé selon la revendication 2, caractérisé en ce que l'agent de condensation est le mésitylène sulfonyl-1 nitro-3 tiazole-1,2,4 (MSNT).

5. Procédé selon la revendication 2, caractérisé en ce que

- l'on déprotège les groupements o-chloro-phényles par une solution de nitro-4 benzaldoxime et de triéthylamine,
- l'on élimine les groupes benzoyles par de l'ammoniaque
- l'on hydrolyse le groupe monométhoxytrityle par de l'acide acétique.

6. Médicament caractérisé en ce qu'il contient un dinucléoside monophosphate tel que spécifié dans la revendication 1.

7. Composition pharmaceutique caractérisée ce ce qu'elle contient un dinucléoside monophosphate tel que spécifié dans la revendication 1 en association avec tout excipient approprié.

Revendications pour l'état contractant : AT

1. Procédé de préparation de dinucléosides monophosphates de formules Ara A-p-Ara C, Ara C-p-Ara A et Ara A-p-Ara A dans lesquelles
Ara A est la 9-β-D-arabinofuranosyl-adénine
Ara C est la 1-β-D-arabinofuranosyl-cytosine
p est le groupe monophosphate,
procédé caractérisé en ce que
1) on prépare les synthons adénine et cytosine portant un groupe OH libre en 3',
2) on prépare les synthons adénine et cytosine portant un groupe OH libre en 5',
3) on effectue la synthèse des dinucléosides monophosphates
- en phosphorylant le synthon portant un OH libre en 3' à l'aide d'un agent phosphorylant bifonctionnel,
- en condensant le diester obtenu avec le nucléoside choisi à l'aide d'un agent de condensation,
- et en déprotégeant les composés obtenus pour arriver aux dimères cherchés sous la forme de sels de triéthylammonium.

2. Procédé selon la revendication 1, caractérisé en ce que l'agent phosphorylant bifonctionnel est l'o-chloro-phényl phosphorodichloridate.

3. Procédé selon la revendication 1, caractérisé en ce que l'agent de condensation est le mésitylène sulfonyl-1 nitro-3 tiazole-1,2,4 (MSNT).

4. Procédé selon la revendication 1, caractérisé en ce que
- l'on déprotège les groupements o-chloro-phényles par une solution de nitro-4 benzaldoxime et de triéthylamine,
- l'on élimine les groupes benzoyles par de l'ammoniaque
- l'on hydrolyse le groupe monométhoxytrityle par de l'acide acétique.

0121635

Office européen des brevets

# RAPPORT DE RECHERCHE EUROPEENNE

Numéro de la demande

EP 83 40 2289

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| Y,D | PROGR. ANTIMICR. & ANTI. CHEM., vol. II,1970, pages 180-184, Tokyo, JP; M. PRIVAT DE GARILHE et al.: "Activité de certains arabinosides et de leurs associations sur le développement de la tumeur ascitique d'Ehrlich chez la souris" * Pages 180-181 * --- | 1,6 | C 07 H 19/20<br>A 61 K 31/70 |
| Y | CHEMICAL ABSTRACTS, vol. 89, no. 21, 20 novembre 1978, page 235, no. 175562z, Columbus, Ohio, USA; PRAKASH BHUTA: "Inhibition of ribosomal peptidyltransferase with cytidylyl-3' - 5'-/2'(3')-O-L-phenylalanyl/-L-adenosine" & BIOCHEM. BIOPHYS. RES. COMMUN. 1978, 83(2), 414-420 * Résumé * --- | 1,6 | DOMAINES TECHNIQUES RECHERCHES (Int. Cl. 3) |
| Y | CHEMICAL ABSTRACTS, vol. 87, no. 23, 5 décembre 1977, page 224, no. 179734p, Columbus, Ohio, USA; MOSHE D. WHITE et al.: "Inhibition of pancreatic ribonuclease by 2'-5' and 3'-5' oligonucleotides" & NUCLEIC ACIDS RES. 1977, 4(9), 3029-3038 * Résumé * --- -/- | 1,6 | C 07 H 19/00<br>A 61 K 31/00 |

Le présent rapport de recherche a été établi pour toutes les revendications

| Lieu de la recherche | Date d'achèvement de la recherche | Examinateur |
|---|---|---|
| LA HAYE | 26-03-1984 | VERHULST W. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503.03.82

0121635

Office européen des brevets

# RAPPORT DE RECHERCHE EUROPEENNE

Numéro de la demande

EP 83 40 2289

Page 2

| DOCUMENTS CONSIDERES COMME PERTINENTS | | | |
|---|---|---|---|
| Catégorie | Citation du document avec indication, en cas de besoin, des parties pertinentes | Revendication concernee | CLASSEMENT DE LA DEMANDE (Int. Cl. 3) |
| Y | JOURNAL OF MEDICINAL CHEMISTRY, vol. 10, no. 4, septembre 1967, pages 777-782; H.E. RENIS et al.: "Nucleic acids. III. Antiviral activity of nucleotides and dinucleoside phosphates containing ara-cytidine" * Pages 777,778 * | 1,2 | |
| Y | CHEMICAL ABSTRACTS, vol. 74, no. 13, 29 mars 1971, page 207, no. 63035p, Columbus, Ohio, USA; M. PRIVAT DE GARILHE et al.: "Effect of some arabinosides and their associations on Ehrlich ascites tumor development in mice" & PROGR. ANTIMICROB. ANTICANCER CHEMOTHER., PROC. INT. CONGR. CHEMOTHER., 6th 1969 (Pub. 1970), 2, 180-184 * Abrégé * | 1,6 | DOMAINES TECHNIQUES RECHERCHES (Int. Cl. 3) |

Le présent rapport de recherche a été établi pour toutes les revendications

| Lieu de la recherche | Date d'achèvement de la recherche | Examinateur |
|---|---|---|
| LA HAYE | 26-03-1984 | VERHULST W. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82